**Europäisches Patentamt**

**European Patent Office**

**Office européen des brevets**

(19)

(11) Veröffentlichungsnummer : **0 374 618 B1**

(12) **EUROPÄISCHE PATENTSCHRIFT**

(45) Veröffentlichungstag der Patentschrift :
**14.04.93 Patentblatt 93/15**

(21) Anmeldenummer : **89122648.2**

(22) Anmeldetag : **08.12.89**

(51) Int. Cl.⁵ : **A61M 5/142**, A61M 5/168,
G01B 21/14

(54) Infusionspumpe.

(30) Priorität : **16.12.88 DE 3842404**

(43) Veröffentlichungstag der Anmeldung :
**27.06.90 Patentblatt 90/26**

(45) Bekanntmachung des Hinweises auf die
Patenterteilung :
**14.04.93 Patentblatt 93/15**

(84) Benannte Vertragsstaaten :
**DE ES FR GB IT**

(56) Entgegenhaltungen :
**CH-A- 646 517**
**DE-A- 1 491 770**
**DE-A- 2 555 291**
**DE-A- 3 326 784**
**US-E- 30 088**

(73) Patentinhaber : **Fresenius AG**
**Gluckensteinweg 5**
**W-6380 Bad Homburg v.d.H. (DE)**

(72) Erfinder : **Polaschegg, Hans-Dieter**
**Grünwiesenweg 9**
**W-6370 Oberursel 4 (DE)**
Erfinder : **Kroh, Robert**
**Pfaffenwiesbacherstrasse 27a**
**W-6393 Wehrheim 1 (DE)**
Erfinder : **Krick, Gerd, Dipl.-Ing. Dr.**
**Kaiser-Friedrich-Promenade 92**
**W-6380 Bad Homburg (DE)**

(74) Vertreter : **Dr. Fuchs, Dr. Luderschmidt Dr.**
**Mehler, Dipl.-Ing Weiss Patentanwälte**
**Abraham-Lincoln-Strasse 7**
**W-6200 Wiesbaden (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 374 618 B1

## Beschreibung

Die Erfindung betrifft eine Infusionspumpe mit einem Förderschlauch aus flexiblem Material wie Kunststoff oder Gummi.

Die moderne Intensivbehandlung von schwerkranken Patienten macht die präzise Zufuhr von Arzneimitteln sowie von für die parenterale Zufuhr geeigneter Nahrung in den Blutkreislauf erforderlich. In weniger kritischen Fällen, zum Beispiel bei reiner Flüssigkeitszufuhr nach Operationen, bedient man sich der sog. Schwerkraftinfusion. Dabei wird die Infusionsrate durch manuelle Veränderung des Strömungswiderstandes einer Infusionsleitung mit Hilfe beispielsweise einer Rollklemme eingestellt. Die Infusionsrate wird durch Bestimmung der Tropfrate in der Tropfkammer des Infusionsgerätes bestimmt.

Eine etwas größere Genauigkeit erreicht man mit sog. Infusionsreglern. Dabei wird gleichfalls die Schwerkraft zur Förderung benutzt, jedoch wird der Strömungswiderstand durch eine geeignete, automatische Einrichtung so geregelt, daß eine vorbestimmte Tropfrate eingehalten wird.

Reicht allerdings die Schwerkraft nicht zur Überwindung des Strömungswiderstandes der angeschlossenen Leitungen, z.B. aufgrund von eingebauten Filtern oder Kathetern, so müssen sog. Infusionspumpen eingesetzt werden.

Hier unterscheidet man im wesentlichen tropfengeregelte Pumpen, volumetrische Pumpen und Spritzenpumpen.

Tropfengeregelte Pumpen haben den grundsätzlichen Nachteil, daß aufgrund des Prinzips eine präzise Förderung eines Wirkstoffes im Sinne der Vorhersagbarkeit der Zufuhr nicht möglich ist, da das Tropfenvolumen in Abhängigkeit von der physikalischen Eigenschaft des zu fördernden Mediums Schwankungen bis zu 400 % unterliegt. Dieses Tropfenvolumen wird hauptsächlich von der Oberflächenspannung beeinflußt, die sich ändern kann, wenn ein Arzneimittel einer Trägerlösung zugesetzt wird.

Volumetrische Infusionspumpen und Spritzenpumpen verfügen in der Regel über elektronisch geregelte Fördermechanismen, deren Abweichungen vom Sollwert unterhalb 5 % liegen. Die Genauigkeit dieser Pumpen wird durch die Genauigkeit des Einmalartikels bestimmt. Bei Spritzenpumpen ist dies der Innendurchmesser der Spritze, bei den peristaltisch arbeitenden Pumpen der Innendurchmesser des Förderschlauchs.

Aufgrund der großen Genauigkeit der für Spritzenpumpen eingesetzten Spritzen werden effektive Genauigkeiten von etwa 2 % erreicht. Bei peristaltischen Pumpen hingegen liegt die Genauigkeit auch bei Verwendung qualitativ hochwertiger Schläuche aufgrund der Toleranz des Innendurchmessers in der Größenordnung von 5 bis 10 %.

Während Spritzenpumpen üblicherweise im Förderratenbereich von < 1 bis 100 ml/h mit einer Auflösung von 0,1 ml/h arbeiten, erlauben peristaltische Infusionspumpen bestenfalls einen Förderbereich von 1 ml/h bis maximal 1.000 ml/h mit einer Auflösung von 1 ml/h. Dabei ist zu berücksichtigen, daß bei kleinen Raten (typisch < 10 ml/h) die Förderratenschwankungen bei kurzen Beobachtungszeiten außerordentlich hoch sind, und dabei solche Pumpen bei kleinen Förderraten nicht für die Applikation von Medikamenten mit kurzer Halbwertszeit im menschlichen Körper (z. B. blutdurckregelnde Medikamente, Anästhetika) geeignet sind.

Diese Ratenschwankungen sind bedingt durch die Periodizität der Peristaltik. Diese wirkt sich um so gravierender aus, je größer das Fördervolumen eines Pumpzyklus ist (d.h. eine Umdrehung des Pumpenkopfes bei einer Rotorperistaltikpumpe bzw. einer vollständigen Sequenz bei einer Linearperistaltik), d.h. je größer der Innendurchmesser des Pumpschlauches ist.

Verringert man den Schlauchdurchmesser, beispielsweise um den Faktor 3,15, so verringert sich der Pumpenschlauchquerschnitt und damit die Förderrate bei gleicher Umdrehungszahl um den Faktor 10. Betreibt man eine peristaltische Pumpe mit einem solchen Schlauch verringerten Innendurchmessers, so wird die Förderratenschwankung bei kleinen Raten und festem Beobachtungsintervall um den Faktor 10 verringert.

Es sind peristaltische Pumpen für medizinische Zwecke bekannt, die die Verwendung unterschiedlicher Einmalartikel erlauben und über Einrichtungen verfügen, die eine automatische Erkennung der Art des Einmalartikels erlauben.

Aus der DE-PS 33 26 784 ist eine mechanische Codierung des Einmalartikels bekannt. Diese Codierung setzt aber voraus, daß sowohl die Pumpe als auch der Einmalartikel über entsprechende spezielle Einrichtungen verfügen müssen. Dies hat den Nachteil, daß ein zusätzlicher Aufwand erforderlich ist, wobei trotzdem nicht die gewünschte und erforderliche Sicherheit erzielt wird, da es immer wieder zu Verwechslungen, auch bei der Fertigung von Einmalartikeln, kommt, wie z. B. das Medical Device Reporting Register der Food and Drug Administration zeigt.

Aus dem DE-U-17 10 902 ist ein Meßgerät zur fortlaufenden Messung des Durchmessers von Schläuchen bekannt. Hierzu wird der zu vermessende Schlauch durch einen in zwei Walzen gebildeten Spalt hindurchgeführt und dort zusammengedrückt. Die Breite des zusammengedrückten Schlauches wird mittels zweier Scheiben bestimmt, die die Form von archimetischen Spiralen besitzen. Aufgrund der vorgegebenen, festgelegten

EP 0 374 618 B1

Spaltbreite ist das Meßergebnis ungenau. Darüber hinaus ist eine Bestimmung des Innendurchmessers des Schlauches nicht möglich.

Aus der DE-C-263 796 ist eine Vorrichtung zur Bestimmung der Weite von schlauchartigen Körpern bekannt, wobei der Schlauch durch zwei in den Schlauch eingeführte Stangen gedehnt wird. Mit dieser Vorrichtung können nur dünnwandige Schläuche mit größeren Durchmesser vermessen werden. Die Vorrichtung ist mechanisch aufwendig und mit einem großen Fehler behaftet.

Aus der DE-A-21 48 976 (US-E-30088) ist eine Vorrichtung zur Aufnahme der Querschnittsdimensionen von Metallrohren bekannt, wobei eine Ultraschalleinrichtung eingesetzt wird. Der Innendurchmesser wird aus dem gemessenen Außendurchmesser sowie der gemessenen Wandstärken ermittelt. Die Metallrohre werden nach Herstellung und vor dem Befüllen oder dem Einbau in eine Anlage zu Kontrollzwecken vermessen.

Ein Sicherheitsinfusionssystem mit einer Rollenpumpe ist bekannt geworden aus der DE-A-14 91 770. Allerdings gibt diese Druckschrift keinerlei Auskunft darüber, ob und ggf. wie der Innendurchmesser von Schläuchen zum Patienten gemessen wird.

Aufgabe der Erfindung ist es, eine Infusionspumpe anzugeben, deren Fördergenauigkeit und Sicherheit verbessert wird.

Die mechanische Einrichtung weist mindestens eine Andruckeinrichtung auf, die senkrecht zur Schlauchachse angreift und im wesentlichen aus einem beweglichen Stempel und einer Widerlagerfläche besteht. Dieser bewegliche Stempel dient zum Zusammendrücken des zu messenden Schlauches, bis die Innenwände des Schlauches aneinanderliegen. Diese Andruckeinrichtung ist an eine Meß- und Auswerteeinheit angeschlossen, die zur Erfassung des vom Stempel zurückgelegten Weges ausgelegt ist.

Unter Berücksichtigung der Dicke der Schlauchwandung kann dann anhand des vom Stempel zurückgelegten Weges der Innendurchmesser des Schlauches ermittelt werden.

Da aber auch die Wanddicke des Schlauches nicht unerheblichen Toleranzen unterliegt, kann eine höhere Genauigkeit gemäß einer weiteren Ausführungsform dadurch erreicht werden, daß die Met- und Auswerteeinheit zur Aufnahme eines Kraft-Weg-Diagrammes ausgelegt ist. ,hierbei wird die Kraft des Stempels beim Zusammendrücken des Schlauches gemessen. Sobald der Stempel an der Außenwand des zu messenden Schlauches angreift, steigt die erforderliche Kraft linear an, bis die Innenwände des Schlauches aneinanderliegen. Ab diesem Punkt nimmt die Steigung der Kraft-Weg-Geraden noch einmal zu. Aus dem Wegabschnitt, der durch Anfang und Ende des linearen Kraftanstieges bestimmt ist, wird der Innendurchmesser des Schlauches ermittelt.

Gemäß einer besonderen Ausführungsform ist der Stempel ein Finger einer linear peristaltisch arbeitenden Pumpe. Dies hat den Vorteil, daß keine zusätzliche Meßvorrichtung an den Schlauch angeschlossen werden mut. Lediglich ein Finger der Peristaltik mut zur Kraft-Weg-Messung ausgelegt sein, wobei die erforderliche Auswertung durch eine in der peristaltischen Pumpe integrierte Auswerteeinheit vorgenommen werden kann. Diese Anordnung bietet weiterhin den Vorteil, daß bei jedem Pumpzyklus der Innendurchmesser des Pumpschlauches kontrolliert werden kann, so daß zusammen mit der Zyklusfrequenz eine ständige Überwachung des Volumenstromes möglich ist. Auf diese Weise wird eine sehr hohe Fördergenauigkeit bei der Verwendung von Standardinfusionsgeräten erreicht.

Anstelle einer Kraft-Weg-Messung kann gemäß einer weiteren Ausführungsform auch eine Zwei-Weg-Messung vorgenommen werden. In diesem Fall ist ein zusätzlicher Meßfinger vorgesehen, der senkrecht zur Schlauchachse und senkrecht zur Bewegungsrichtung des Stempels beweglich angeordnet ist. Mit Hilfe dieses Meßfingers, der ebenfalls an die Meß- und Auswerteeinheit angeschlossen ist, wird der maximale Außendurchmesser des durch den Stempel zusammengedrückten Schlauches ermittelt. Aus den beiden Meßgrößen a und b, die nach dem Zusammendrücken des Schlauches den minimalen Außendurchmesser bzw. den vom Meßfinger gemessenen maximalen Außendurchmesser des deformierten Schlauches bezeichnen wird der Innendurchmesser d nach folgender Formel ermittelt:

$$d = (b - a) \times 2/\Pi$$

Die Ermittlung der Maße a und b sowie die Berechnung kann auf verschiedene Arten geschehen: mechanisch, optisch, hydraulisch oder elektrisch bzw. aus Kombinationen dieser Meßverfahren.

Gemäß einer bevorzugten Ausführungsform können Stempel und Meßfinger mit je einem Kolben eines hydraulischen Systems verbunden sein, so daß durch geeignete Wahl der Kolbenflächen und der durch die Kolben verdrängten Flüssigkeitsmenge der Innendurchmesser durch eine entsprechende Anzeigevorrichtung direkt angezeigt werden kann.

Gemäß einer weiteren Ausführungsform sind Stempel und Meßfinger an eine elektrische Meßbrücke angeschlossen, die im wesentlichen zwei Potentiometer aufweist.

Anstatt auf mechanischem Wege den Innendurchmesser des Schlauches zu ermitteln, wird gemäß einer anderen Lösung derselben Aufgabe eine Ultraschalleinrichtung verwendet, die mindestens einen an der Außenseite des Pumpenschlauches anliegenden Ultraschallsender und mindestens einen ebenfalls an der Au-

3

ßenseite des Pumpenschlauches anliegenden Ultraschalldetektor aufweist, die beide an eine Meß- und Auswerteeinrichtung angeschlossen sind, die zur Bestimmung des Innendurchmessers des Schlauches aus einer Lauf-Zeit-Messung ausgebildet ist.

Die Anordnung von Ultraschallsender und Ultraschallempfänger kann auf verschiedene Weise erfolgen. So können Sender und Empfänger gegenüberliegen oder aber die Sender und Empfänger können derart angeordnet sein, daß eine Lauf-Zeit-Messung unter einem Reflexionswinkel > 0° durchführbar ist. Vorzugsweise wird die Reflexionslaufzeitmessung unter einem Reflexionswinkel von 45° durchgeführt.

Ultraschallsender und Ultraschallempfänger können auch in einem gemeinsamen Sensor kombiniert sein, wobei in diesem Fall der Reflexionswinkel 180° beträgt.

Da peristaltische Infusionspumpen in der Lage sind, einen Unterdruck auf der Saugseite zu erzeugen, müssen diese daher stromab über einen Luftdetektor verfügen. Da Luftdetektoren üblicherweise ebenfalls nach dem Ultraschallprinzip arbeiten, kann gemäß einer weiteren bevorzugten Ausführungsform die Ultraschalleinrichtung zur Bestimmung des Innendurchmessers von Schläuchen aus einem derartigen Ultraschalluftdetektor bestehen, wobei die Auswerteeinheit zusätzlich zur Bestimmung des Innendurchmessers des Schlauches aus Lauf-Zeit-Messungen ausgebildet ist.

Mit Hilfe der erfindungsgemäßen Vorrichtung ist die Ermittlung des Schlauchinnendurchmessers unmittelbar vor, nach oder im Pumpbereich einer Infusionspumpe möglich, wobei beispielsweise bei peristaltischen Pumpen ohne großen technischen Mehraufwand der Innendurchmesser des Pumpschlauches und damit die Förderleistung der peristaltischen Pumpe permanent kontrolliert werden kann, so daß auch Veränderungen des Innendurchmessers des Schlauches aufgrund von Ermüdungserscheinungen des Schlauchmaterials während des Pumpvorgangs erkannt werden können.

Beispielhafte Ausführungsformen der Erfindung werden nachfolgend anhand der Zeichnung näher erläutert.

Es zeigen:

| | |
|---|---|
| Figur 1 | eine perspektivische Ansicht der Einrichtung zur Messung des Innendurchmessers gemäß einer Ausführungsform, |
| Figur 2 | einen Schnitt durch die Einrichtung gemäß Figur 1, |
| Figur 3 | die schematische Darstellung eines hydraulischen Meßsystems, |
| Figur 4 | die schematische Darstellung eines elektrischen Meßsystems, |
| Figur 5 | ein Kraft-Weg-Diagramm, wie es beim Zusammendrücken eines Schlauches auftritt, |
| Figuren 6a, b | jeweils eine schematische Darstellung einer linear peristaltisch arbeitenden Pumpe gemäß zweier weiterer Ausführungsformen, |
| Figur 7 | eine Ultraschalleinrichtung gemäß einer ersten Ausführungsform und |
| Figur 8 | eine Ultraschalleinrichtung gemäß einer weiteren Ausführungsform. |
| Figur 9 a | eine Rollenpumpe mit Ultraschalleinrichtung |
| Figur 9 b | eine linear peristaltisch arbeitende Pumpe mit vorgeschalteter mechanischer Einrichtung zur Messung des Innendurchmessers. |

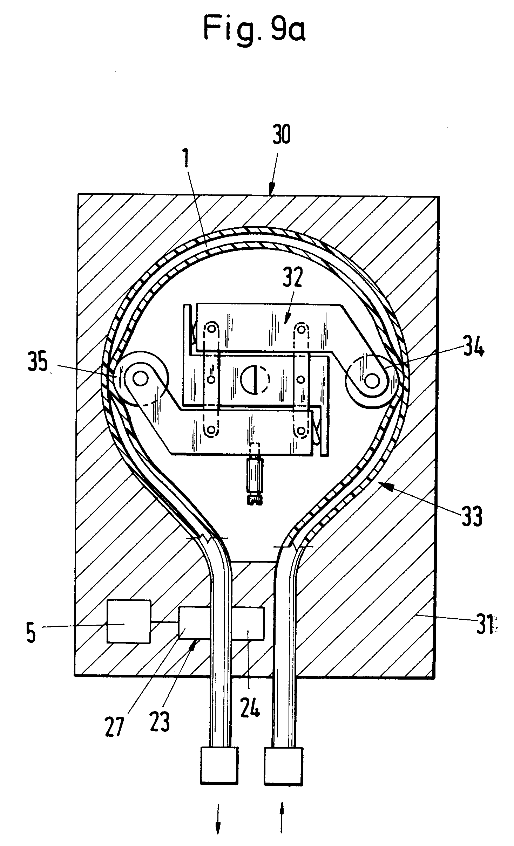

In der Figur 9a ist eine peristaltisch arbeitende Rollenpumpe 30 dargestellt, die ein Statorteil 31 und einen Rotor 32 aufweist. Im Statorteil 31 ist ein Pumpenbett 33 zur Aufnahme des Pumpenschlauches 1 ausgebildet. Am Rotor 32 sind Rollen 34, 35 drehbeweglich gelagert, die gegen den Pumpenschlauch 1 drücken.

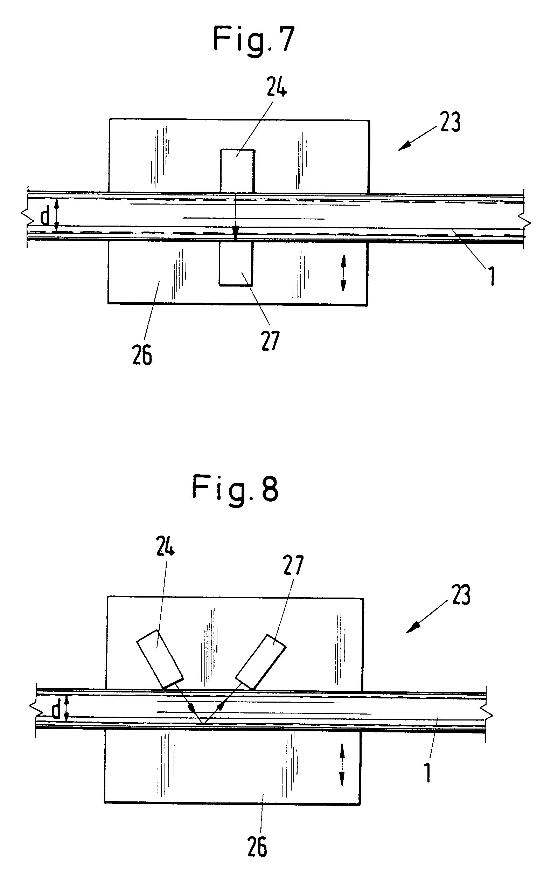

Im unteren Teil des Stators 31 im Bereich des ausgangsseitigen Endes des Schlauches 1 ist eine Ultraschalleinrichtung 23 zur Messung des Innendurchmessers des Schlauches 1 angeordnet. Die Ultraschalleinrichtung 23 weist einen Sender 24, einen Empfänger 27 sowie eine Meß- und Auswerteeinrichtung 5 auf. Zwei mögliche Ausführungsformen der Ultraschalleinrichtung sind in den Figuren 7 und 8 dargestellt.

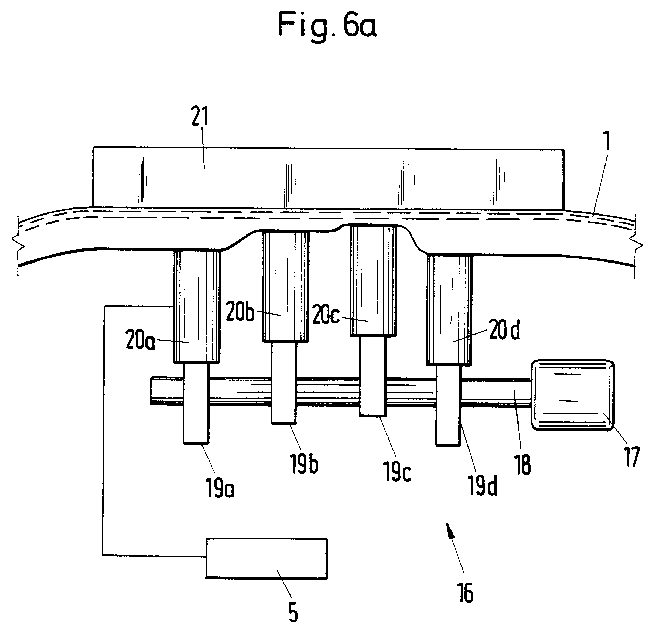

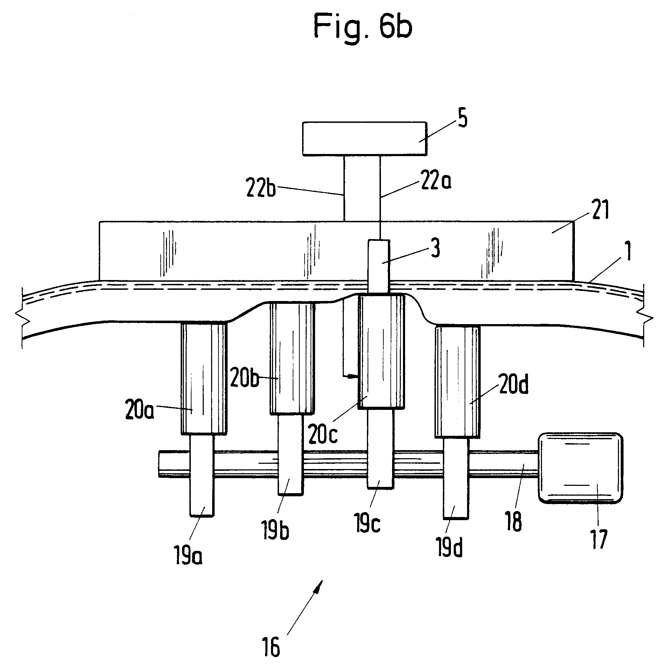

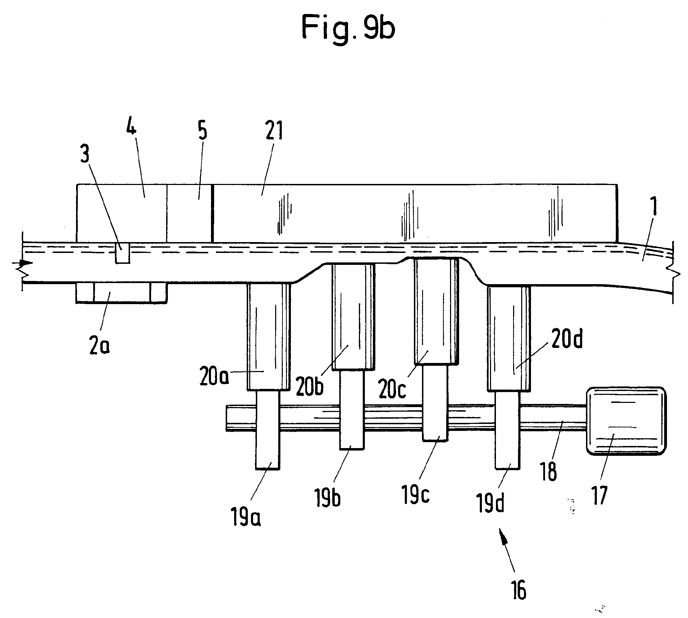

In der Figur 9b ist eine linear peristaltisch arbeitende Pumpe 16 schematisch dargestellt, die im Zusammenhang mit den Figuren 6a und 6b noch eingehender beschrieben wird. Am Pumpeneingang ist eine mechanische Einrichtung zur Messung des Innendurchmessers des Schlauches 1 angeordnet, die ein winkelförmiges Gehäuse 4 und einen beweglichen Stempel 2a, einen Meßfinger 3 sowie eine Auswerteeinrichtung 5 aufweist.

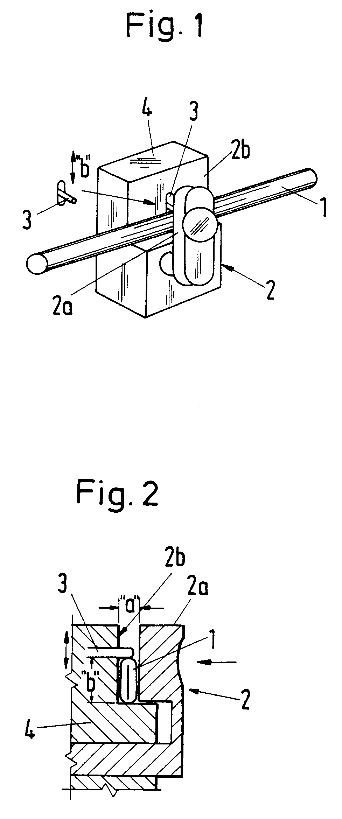

In der Figur 1 ist in perspektivischer Darstellung die Einrichtung zur Messung des Innendurchmessers des Schlauches 1 dargestellt. Das winkelförmige Gehäuse 4 besitzt innenliegende Winkelflächen zur Aufnahme des zu messenden Schlauches 1 dienen. Die senkrecht stehende Winkelfläche 2b dient als Widerlagerfläche und ist parallel zur ebenen Fläche des Stempels 2a der Andruckeinrichtung 2 angeordnet. Dieser Stempel 2a ist beweglich im Gehäuse angeordnet und mit einem Antrieb verbunden, so daß der Stempel in Richtung auf die Widerlagerfläche 2b bewegt werden kann, wobei der Schlauch 1 in der Weise deformiert wird wie in Figur 2 zu sehen ist. In der Widerlagerfläche 2b ist der Meßfinger 3 angeordnet, der während der Deformation des Schlauches 1 durch den Stempel 2a nach oben ausgelenkt wird. Wenn der Schlauch 1 vollständig deformiert ist, wird aus dem zurückgelegten Weg des Stempels 2a der Abstand a zwischen der Stempelfläche und der Widerlagerfläche 2b ermittelt. Gleichzeitig wird die Auslenkung b des Meßfingers 3 gemessen und in der im Gehäuse 4 untergebrachten Meß- und Auswerteeinheit nach der Formel (b-a) x $2/\pi$ der Innendurchmesser des Schlauchs 1 ermittelt.

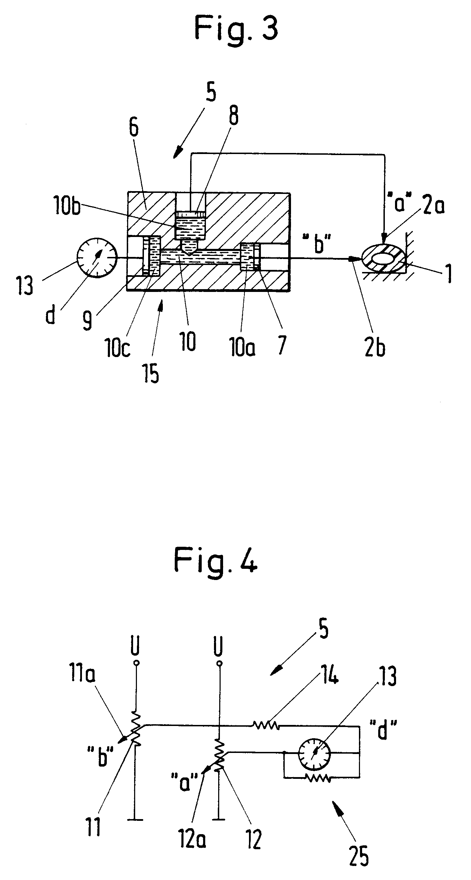

In der Figur 3 ist schematisch ein hydraulisches Meßsystem dargestellt. Der Schlauch 1 wird auch in diesem Fall von dem Stempel 2a deformiert, wobei der maximale Außendurchmesser nach der Deformation von dem Meßfinger 2b gemessen wird. Stempel 2a und Meßfinger 2b sind in der Figur 3 durch die jeweiligen Pfeile angedeutet. Der Stempel 2a und der Meßfinger 2b sind mit jeweils einem Kolben 8 bzw. 7 eines hydraulischen Systems verbunden. Diese Kolben 7 und 8 sind in entsprechend dimensionierten Endabschnitten 10a bzw. 10b eines gemeinsamen Kanales 10 angeordnet, der über einen dritten Kanalabschnitt 10c mit einem weiteren Kolben 9 in Verbindung steht, der an ein Anzeigegerät 13 angeschlossen ist. Durch geeignete Wahl der Kolbenflächen wird der Faktor $2/\pi$ bei der Anzeige berücksichtigt.

In der Figur 4 ist eine Meß- und Auswerteeinheit 5 dargestellt, die aus einer Meßbrücke 25 besteht. Stempel 2a und Meßfinger 3 sind an jeweils einen Potentiometer 12 bzw. 11 angeschlossen. Aufgrund der durch die Stellung des Meßfingers 3 und des Stempels 2a unterschiedlichen Positionierung des jeweiligen Abnehmers 11a bzw. 12a der Potentiometer 11 und 12 wird eine Spannung an den Abnehmern abgegriffen, die als Innendurchmesser d auf der Anzeigeeinrichtung 13 abgelesen werden kann. Durch geeignete Dimensionierung der Widerstände 11, 12, 14 wird der Faktor $2/\pi$ berücksichtigt.

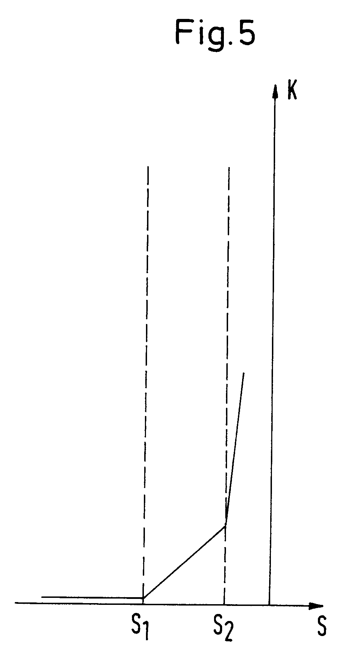

Ist der Stempel 2a mit einem Kraft-Weg-Aufnehmer ausgerüstet und ist die Auswerteeinheit zur Auswertung eines Kraft-Weg-Diagrammes ausgelegt, so kann auf den Meßfinger 3 verzichtet werden. In der Figur 5 ist ein solches Kraft-Weg-Diagramm dargestellt. Solange der Stempel noch nicht an der Außenseite des Schlauches 1 anliegt, ist die aufzuwendende Kraft nahe 0. Sobald der Stempel an der Außenwand des Schlauchs 1 angreift, dies ist der Punkt S1 in der Figur 5, steigt die aufzuwendende Kraft linear an bis ein Punkt S2 erreicht ist, an dem die Innenwände des Schlauchs 1 aufeinanderliegen. Der nachfolgende Kraft-Weg-Verlauf wird durch die elastischen Eigenschaften der Schlauchwand bestimmt. Dementsprechend ist jetzt auch ein größerer Kraftaufwand erforderlich, was sich in einer größeren Steigung der Kraft-Weg-Kurve ausdrückt. Der Wegabschnitt zwischen S1 und S2 gibt dann direkt den Innendurchmesser des gemessenen Schlauchs 1 an.

In den Figuren 6a und 6b sind zwei weitere Ausführungsformen dargestellt, die bei einer linear peristaltisch arbeitenden Pumpe 16 zur Anwendung kommen. In der Figur 6a ist eine solche linear peristaltisch arbeitende Pumpe schematisch dargestellt. Über eine gemeinsame Antriebswelle 18, die mit einem Antriebsmotor 17 verbunden ist, werden über Kurvenscheiben 19a bis 19d die Pumpstößel 20a bis 20d in einer solchen Weise gegen den Schlauch 1 gedrückt, daß der Schlauch in Form einer peristaltischen Bewegung deformiert wird. Als Widerlager dient eine Platte 21, an der der Schlauch 1 anliegt. In der hier gezeigten Ausführungsform ist der Pumpstößel 20a mit einem Kraft-Weg-Aufnehmer ausgerüstet, der an eine entsprechende Meß- und Auswerteeinheit 5 angeschlossen ist. Durch die Auswertung des Weg-Zeit-Diagramms, wie es in der Figur 5 dargestellt ist, wird der Innendurchmesser ermittelt. Der Vorteil dieser Einrichtung besteht darin, daß bei jedem Pumpzyklus der Innendurchmesser des Schlauches 1 gemessen werden kann, so daß eine permanente Überwachung der Förderrate der peristaltischen Pumpe 16 möglich ist.

In der Figur 6b ist die gleiche peristaltische Pumpe 16 dargestellt, jedoch weist hier der Pumpstößel 20c keinen Kraft-Weg-Aufnehmer auf. Die Funktionsweise der hier gezeigten Vorrichtung entspricht der in der Figur 1 gezeigten Anordnung. Der Pumpstößel 20c bewirkt die Deformation des Schlauches 1. Die Andruckplatte 21 weist im Bereich des Pumpenstößels 20c den Meßfinger 3 auf, der während der Deformation des Schlauches 1, wie bereits zu den Figuren 1 und 2 beschrieben, ausgelenkt wird. Der Pumpenstößel 20c ist über die elektrische Leitung 22b mit der Auswerteeinheit 5 und der Meßfinger 3 über die elektrische Leitung 22a ebenfalls mit der Auswerteeinheit 5 verbunden.

In der Figur 7 ist die Ultraschalleinrichtung 23 dargestellt, die aus dem Sender 24 und dem Empfänger 27 sowie einer hier nicht gezeigten Meß- und Auswerteeinrichtung besteht. In dem Ausführungsbeispiel der Figur 7 ist der Sender dem Empfänger gegenüber angeordnet. Der Empfänger befindet sich auf einer verschiebbaren Platte 26, die nach Einlegen des Schlauchs 1 verschoben werden kann, so daß der Empfänger an der Außenwand des zu messenden Schlauchs 1 anliegt. Aus der Laufzeit des Ultraschallsignals zwischen dem Sender 24 und dem Empfänger 27 wird in der Meß- und Auswerteeinheit 5 der Innendurchmesser d des Schlauchs 1 bestimmt, wobei die Wanddicke des Schlauchs 1 berücksichtigt werden muß.

In der Figur 8 sind der Ultraschallsender 24 und der Ultraschallempfänger 27 in Reflexionsstellung angeordnet. In dem hier gezeigten Ausführungsbeispiel wird die Ultraschallwelle an der Innenwand des Schlauches 1 unter einem Winkel von etwa 45° reflektiert.

Bezugszeichenliste

1          Schlauch

| | |
|---|---|
| 2 | Andruckeinrichtung |
| 2a | Stempel |
| 2b | Widerlagerfläche |
| 3 | Meßfinger |
| 4 | Gehäuse |
| 5 | Meß- und Auswerteeinrichtung |
| 6 | Meßblock |
| 7 | Kolben |
| 8 | Kolben |
| 9 | Kolben |
| 10 | gemeinsamener Flüssigkeitskanal |
| 10a,b,c | Endabschnitte des Flüssigkeitskanals |
| 11 | Potentiometer |
| 11a | Abnehmer |
| 12 | Potentiometer |
| 12a | Abnehmer |
| 13 | Anzeigeeinrichtung |
| 14 | Widerstand |
| 15 | hydraulische Meßeinrichtung |
| 16 | linear peristaltisch arbeitende Pumpe |
| 17 | Motor |
| 18 | Antriebswelle |
| 19a,b,c,d | Kurvenscheiben |
| 20a,b,c,d | Pumpenstößel |
| 21 | Andruckplatte |
| 22a,b | elektrische Leitungen |
| 23 | Ultraschalleinrichtung |
| 24 | Sender |
| 25 | Meßbrücke |
| 26 | Platte |
| 27 | Ultraschallempfänger |

**Patentansprüche**

1.  Infusionspumpe mit einem Förderschlauch aus flexiblem Material wie Kunststoff oder Gummi, dadurch gekennzeichnet,
    daß die Infusionspumpe eine Einrichtung zur Messung des Innendurchmessers des Förderschlauches (1) aufweist,
    die mindestens eine senkrecht zur Schlauchachse angreifende Andruckeinrichtung (2) mit einem beweglichen Stempel (2a) und einer Widerlagerfläche (2b) zum vollständigen Zusammendrücken des Förderschlauchs (1), und
    eine an die Andruckeinrichtung (2) angeschlossene Meß- und Auswerteeinrichtung (5) umfaßt, die zur Erfassung des vom Stempel (2a) zurückgelegten Wegs zur Berechnung des Innendurchmessers des Förderschlauchs (1) ausgelegt ist.

2.  Infusionspumpe nach Anspruch 1, dadurch gekennzeichnet, daß der Stempel (2a) eine ebene Fläche aufweist und daß die Widerlagerfläche (2b) parallel zur Stempelfläche angeordnet ist.

3.  Infusionspumpe nach Anspruch 1 oder 2, dadurch gekennzeichnet, daß die Meß- und Auswerteeinrichtung (5) zur Aufnahme eines Kraft-Weg-Diagrams der Andruckeinrichtung und zur anschließenden Bestimmung des Innendurchmessers (d) des Förderschlauchs (1) aus dem Kraft-Weg-Diagramm ausgebildet ist.

4.  Infusionspumpe nach einem der Ansprüche 1 bis 3, dadurch gekennzeichnet, daß mindestens ein senkrecht zur Schlauchachse und zur Bewegungsrichtung der Andruckeinrichtung (2) beweglicher Meßfinger zur Bestimmung des maximalen Außendurchmessers des deformierten Förderschlauchs (1) vorgesehen ist.

5. Infusionspumpe nach einem der Ansprüche 1 bis 4, dadurch gekennzeichnet, daß der Stempel (2a) ein Finger einer linear peristaltisch arbeitenden Pumpe (16) ist.

6. Infusionspumpe nach Anspruch 4, dadurch gekennzeichnet, daß die Meß- und Auswerteeinrichtung (5) aus einem hydraulischen Meßsystem (15) besteht.

7. Infusionspumpe nach Anspruch 4, dadurch gekennzeichnet, daß die Meß- und Auswerteeinrichtung (5) aus einer Widerstandsmeßbrücke (25) besteht.

8. Infusionspumpe mit einem Förderschlauch aus flexiblem Material wie Kunststoff oder Gummi, dadurch gekennzeichnet,
daß die Infusionspumpe eine Einrichtung zur Messung des Innendurchmessers des Förderschlauchs aufweist,
die eine Ultraschalleinrichtung (23) mit mindestens einem an der Außenseite des Förderschlauchs (1) angeordneten Ultraschallsender (24),
mindestens einen ebenfalls an der Außenseite des Förderschlauches (1) angeordneten Ultraschallempfänger (27) und
eine an den Ultraschallsender (24) und den Ultraschallempfänger (27) angeschlossene Meß- und Auswerteeinrichtung (5) umfaßt, die zur Bestimmung des Innendurchmessers des Schlauchs aus einer Lauf-Zeit-Messung ausgebildet ist.

9. Infusionspumpe nach Anspruch 8, dadurch gekennzeichnet, daß der Ultraschallsender (24) und der Ultraschalldetektor (27) gegenüberliegend angeordnet sind.

10. Infusionspumpe nach Anspruch 8, dadurch gekennzeichnet, daß die Meß- und Auswerteeinrichtung (5) zur Laufzeitmessung in Reflexion ausgebildet ist.

11. Infusionspumpe nach Anspruch 10, dadurch gekennzeichnet, daß ein Ultraschallsensor vorgesehen ist, der als Sender und Empfänger dient.

12. Infusionspumpe nach einem der Ansprüche 8 bis 11, dadurch gekennzeichnet, daß der Ultraschallsender und der Ultraschallempfänger (27) derart angeordnet sind, daß eine Laufzeitmessung unter einem Reflexionswinkel von > 0° vorgesehen ist.

13. Infusionspumpe nach einem der Ansprüche 8 bis 12, dadurch gekennzeichnet, daß der Ultraschallsender (24) und der Ultraschallempfänger (27) derart angeordnet sind, daß eine Laufzeitmessung unter einem Reflexionswinkel von 45° durchführbar ist.


## Claims

1. An infusion pump system comprising a supply tube formed from a flexible material such as plastic or rubber, characterized in
that said infusion pump system comprises means for measuring the internal diameter of said supply tube (1)
which comprises at least one counterpressure device (2) arranged perpendicular to the axis of said supply tube and including a movable plunger (2a) and an abutment surface (2b) for complete compression of said supply tube (1), and
a measurement and evaluation unit (5) associated to said counterpressure device (2) for evaluating the distance traversed by said movable plunger (2a) for calculating the internal diameter of said supply tube (1).

2. An infusion pump system according to claim 1, characterized in that the plunger (2a) has a planar surface and that said abutment surface (2b) is parallel to said plunger surface.

3. An infusion pump system according to claim 1 or 2, characterized in that said measurement and evaluation unit (5) is embodied for generating a force-displacement diagram of the counterpressure device and for subsequent determining the internal diameter (d) of said supply tube (1) from said force-displacement diagram.

7

4. An infusion pump system according to any of claims 1 to 3, characterized in that at least one measurement baffle perpendicular to said axis of said supply tube and to the direction of movement of said counterpressure device (2) for measurement of the maximum external diameter of said deformed supply tube (1) is provided.

5. An infusion pump system according to any of claims 1 to 4, characterized in that said plunger (2a) is a finger of a linear peristaltic pump (16).

6. An infusion pump system according to claim 4, characterized in that said measurement and evaluation unit (5) consists of a hydraulic measurement system (15).

7. An infusion pump system according to claim 4, characterized in that said measurement and evaluation unit (5) consists of a resistance measurement bridge (25).

8. An infusion pump system comprising a supply tube formed from a flexible material such as plastic or rubber, characterized in
that said infusion pump system comprises means for measuring the internal diameter of said supply tube (1)
which comprises an ultrasound device (23) including at least one ultrasound transmitter (24) arranged external to said supply tube (1),
at least one ultrasound receiver (27) also arranged external to said supply tube (1) and a measurement and evaluation unit (5) associated with said ultrasound transmitter (24) and said ultrasound receiver (27) and operative for determining said internal diameter of said tube based on a propagation-time-measurement.

9. An infusion pump system according to claim 8, characterized in that said ultrasound transmitter (24) lies opposite said ultrasound receiver (27).

10. An infusion pump system according to claim 8, characterized in that said measurement and evaluation unit (5) is operative for measurement of said propagration-time-measurement in a reflection mode.

11. An infusion pump system according to claim 10, characterized in that an ultrasound sensor is provided which serves as transmitter and receiver.

12. An infusion pump system according to any of claims 8 to 11, characterized in that said ultrasound transmitter and said ultrasound receiver (27) are arranged such that a propagation-time-measurement at a reflection angle of $>0°$ is provided.

13. An infusion pump system according to any of claims 8 to 12, characterized in that said ultrasound transmitter (24) and said ultrasound receiver (27) are arranged such that a propagation-time-measurement at a reflection angle of $45°$ is realizable.

## Revendications

1. Pompe de perfusions pourvue d'un tuyau d'alimentation en matériau flexible tel qu'en matière plastique ou en caoutchouc, caractérisée en ce que la pompe de perfusion présente un dispositif de mesure du diamètre intérieur du tuyau d'alimentation (1), comprenant au moins un dispositif presseur (2) agissant perpendiculairement par rapport à l'axe du tuyau et composé d'un presseur mobile (2a) et d'une face de butée (2b) pour écraser complètement le tuyau d'alimentation (1), et comprenant également un dispositif de mesure et d'évaluation (5) raccordé au dispositif presseur (2) et destiné à déterminer le déplacement du presseur (2a) pour calculer le diamètre intérieur du tuyau d'alimentation (1).

2. Pompe de perfusions selon la revendication 1, caractérisée en ce que le presseur (2a) présente une surface plane et en ce que la face de butée (2b) est disposée parallèlement à la face du presseur.

3. Pompe de perfusions selon la revendication 1 ou 2, caractérisée en ce que le dispositif de mesure et d'évaluation (5) est capable de produire un diagramme puissance/déplacement du dispositif presseur et de déterminer ensuite le diamètre intérieur (d) du tuyau d'alimentation (1) à partir du diagramme puissance/déplacement.

4. Pompe de perfusions selon l'une des revendications 1 à 3, caractérisée en ce qu'est prévu au moins un doigt mesureur mobile, perpendiculaire à l'axe du tuyau et à la direction de déplacement du dispositif presseur (2), pour calculer le diamètre extérieur maximal du tuyau d'alimentation (1) déformé.

5. Pompe de perfusions selon l'une des revendications 1 à 4, caractérisée en ce que le presseur (2a) est un doigt d'une pompe péristaltique linéaire (16).

6. Pompe de perfusions selon la revendication 4, caractérisée en ce que le dispositif de mesure et d'évaluation (5) est constitué d'un système de mesure hydraulique (15).

7. Pompe de perfusions selon la revendication 4, caractérisée en ce que le dispositif de mesure et d'évaluation (5) est constitué d'un pont de résistances de mesure (25).

8. Pompe de perfusions pourvue d'un tuyau d'alimentation en matériau flexible, tel qu'en matière plastique ou en caoutchouc, caractérisée en ce que la pompe de perfusions présente un dispositif de mesure du diamètre intérieur du tuyau d'alimentation comprenant un dispositif à ultra-sons (23) comportant au moins un émetteur d'ultra-sons (24) disposé sur la face extérieure du tuyau d'alimentation (1), au moins un récepteur d'ultra-sons (27) également disposé sur la face extérieure du tuyau d'alimentation (1), et un dispositif de mesure et d'évaluation (5) raccordé à l'émetteur d'ultra-sons (24) et au récepteur d'ultra-sons (27), qui, pour calculer le diamètre intérieur du tuyau, est conformé en mesureur de déplacement/temps.

9. Pompe de perfusions selon la revendication 8, caractérisée en ce que l'émetteur d'ultra-sons (24) et le détecteur d'ultra-sons (27) sont disposés face à face.

10. Pompe de perfusions selon la revendication 8, caractérisée en ce que le dispositif de mesure et d'évaluation (5), destiné à la mesure de déplacement/temps, est conformé en réflexion.

11. Pompe de perfusions selon la revendication 10, caractérisée en ce qu'est prévu un capteur d'ultra-sons servant d'émetteur et de récepteur.

12. Pompe de perfusions selon l'une des revendications 8 à 11, caractérisée en ce que l'émetteur et le récepteur d'ultra-sons (27) sont disposés de telle sorte qu'une mesure de déplacement/temps est prévue selon un angle de réflexion > 0°.

13. Pompe de perfusions selon l'une des revendications 8 à 12, caractérisée en ce que l'émetteur d'ultra-sons (24) et le récepteur d'ultra-sons (27) sont disposés de telle sorte qu'une mesure de déplacement-/temps peut être réalisée selon un angle de réflexion de 45°.

# Fig. 1

# Fig. 2

# Fig. 3

# Fig. 4

# Fig.5

# Fig. 6a

# Fig. 6b

# Fig.7

# Fig.8

# Fig. 9a

# Fig. 9b